# EUROPEAN PATENT APPLICATION

(11) **EP 1 507 227 A2**
(43) Date of publication of application: **16.02.2005**
(21) Application number: 04077872.2
(22) Date of filing: 04.09.2001
(51) Int. Cl.: G06F 19/00

(54) **Improvements relating to Information Management Systems**

(30) Priority: 04.09.2000 GB 0021663; 05.09.2000 GB 0021790
(62) Divisional of application: 01963226.4
(71) Applicant: Enigma Health UK PLC, Cobham Surrey KT11 3EP (GB)
(72) Inventor: Leaman, Maurice, Stanmore, Middlesex HA7 4NW (GB); Saley, Ahmed, Bushey, Hertfordshire WD23 4SG (GB); Shah, Anil, Harrow, Middlesex HA3 9HL (GB)
(74) Representative: Ahmad, Sheikh Shakeel

(57) **Abstract**

A prescription reordering system for requesting remote repeat prescriptions for patients by way of a multistage, multiparty repeat prescription re-ordering process which includes a prescription authorisation stage involving a third party. The system comprises: communications means arranged to receive a request for a repeat prescription via a data network, to enable patient selection of one of a plurality of known pharmacies, to forward the request to the patient-specified pharmacy, and to receive a corresponding response from the pharmacy regarding the allowance of the request; storage means for storing the response to the request from the pharmacy for patient consideration; and status indication means arranged to be updated by the patient-specified pharmacy to indicate to the patient the current position of the request along the multistage repeat prescription process and to specify at least when a request has been submitted to the pharmacy, when the request has been sent to the third party (surgery) for authorisation and when the prescription is ready for delivery or collection.

## Description

### Field of the Invention

The present invention concerns improvements relating to information management systems and more particularly, though not exclusively, to a patient information management system for use by pharmacists in handling repeat prescription requests, enabling patients to view their patient drugs records, to check drug interactions and inform patients of the progress of a prescription request. The present invention also extends to a graphical user interface for use by pharmacists in managing government regulations for account of prescribed drugs.

### Background to the Invention

There are approximately 12,000 community or retail pharmacies in Great Britain of which about 1000 are in Scotland. In order to supply medicines against authorised prescriptions from qualified prescribers (GPs for example), all pharmacy premises and all pharmacists that work in them must be registered with the Royal Pharmaceutical Society of Great Britain (RPSGB).

The overwhelming majority of community pharmacists are contracted into the National Health Service (NHS) a tax-funded government health service. The prescriptions they dispense must be written on NHS docket forms (prescriptions) by the prescribers (doctors for example) who are contracted to the NHS. The NHS remunerates the pharmacists for dispensing these prescriptions and reimburses the costs of the drugs supplied. Pharmacists may also receive non-NHS prescriptions, private prescriptions, from UK registered prescribers and in this case the patient must meet the costs.

Pharmacies are located in nearly every neighbourhood and as such play a pivotal role in the community. The pharmacist is often the first port of call for local people, especially the old and young mothers, and as such the pharmacist regularly performs an extended service over and above that which he is contracted to do and gets paid to do.

Dispensing on average 200 prescriptions a day (with all the attendant professional checks and balances) is time consuming enough and the unpaid extended role often meets resistance from the profession. Testing (blood sugar levels, cholesterol, etc), measuring compliance (using the dispensary PC) and trying to ensure concordance (making sure the patient knows why they are taking the drug and the implications if they don't), all add to a busy and often stressful day. While all this is going on, the owner/manager pharmacist is trying to look after the front of shop sales, manage the staff and all the other things that go with running a successful small business.

The pharmacy is therefore a necessary and essential part of primary healthcare serving the needs of an ever increasing ageing population and the "worried well". It is at the forefront of medicines management and the treatment of minor ailments though this is taking an ever increasing amount of the pharmacists time. The pharmacy is the only viable economic method for the NHS to distribute prescription drugs and with low margins fee and drug margins, mail order, even if it were legal in the UK, would find it difficult to compete on price.

Although a lot of pharmacists take an active interest in their customer's health, there is no formal system or process for doing so. The doctor prescribes and the pharmacist dispenses. Other than returning to the doctor if the desired outcome fails to materialise, there is next to nothing being done to evaluate a patient's progress.

Much has been talked about "medicines management", but little has been done. A recent survey showed that 11% of patients do not even take their prescription and 34% who do start, do not finish! This is high cost waste. For some years now, the profession has argued for 28 day prescribing only. This has not endeared pharmacy to its colleagues in general practice (who saw a longer waiting line outside their surgeries) or to manufacturers whose shareholder duty was in effect to "sell more pills". What this state of affairs does highlight however is a greater need for patient compliance and concordance. Patients do not now fully understand why they are being prescribed a medication and there is no way of ensuring that they take it. Not only is there the cost of the wasted drug but there is also a potentially very expensive time bomb ticking away if the secondary care system has to pick up the tab when a patient's health deteriorates and they are admitted into hospital.

The pharmacy today relies heavily on technology. From the early beginnings of hand held stock re-ordering devices in the seventies to today's on-line Internet pharmacies, the pharmacy has wrestled with new technologies, often resisting change as, unlike GPs, the costs of PCs etc had to be borne by the business and not paid for by the government.

PMRs (Patient Medical Records) necessitated a PC in the dispensary in the mid/late eighties but still pharmacies were slow to exploit the hardware. Often, at best, small business accounting was the only concession to automation. Nowadays, automated pharmacy dispensing systems are known which manage the creation, updating and use of patient drugs records (PDRs). These records are used to assist the pharmacist in data entry and checking for drug interaction for example. The systems also manage the sale of drugs and other items as well as processing prescription data. This is often coupled together with an Electronic Point of Sale terminal.

The systems which exist are on the whole local systems which are designed to assist the pharmacist in carrying out their local tasks and do not need or aspire to support the above described wider patient healthcare management role. Other than postal questionnaires, there are no mechanisms for obtaining feedback on the service that is provided, on the way in which patients react to the prescribed medication and little in the way of support for the patient after their visit to their GP.

It is desired to overcome at least some of the above described problems and to provide an improved system for managing patient information.

### Summary of the Invention

According to a first aspect of the present invention there is provided a patient information management system for enhancing viewing of a patient's personal patient drugs record, the system comprising: communications means arranged to receive patient drugs records created at a pharmacy connectable to the system via a communications link; and a database of the received patient drugs records, each record providing personal information about the patient, which does not reveal patient identity, and being identifiable by a unique pharmacy-assigned identifier; wherein the communications means is also arranged to receive support information from a support organisation, the support information comprising matching requirement data associated therewith for matching the support information with a specific category of patients; and the system further comprises: a matching engine arranged to target the support information to appropriate patients by matching the requirement data with the personal patient information such that any patient access to the drugs record enables provision of targeted support information to the patient.

The patient drug record (PDR) stored in the database, does not reveal patient identity by omitting any information regarding the name, address or direct contact details (e.g. telephone number) of the patient. This means that the PDR is to all intensive purposes anonymous such that use of that record at the system for targeting support information does not compromise patient confidentiality.

The present invention enables use of patients' drug profile and possibly their demographic profile to provide an entirely new and unique series of on-line products that are beneficial to the patient, the GP, the pharmacist, the drug manufacturer and the NHS. It is believed that these are services currently unavailable anywhere in the world. Patients can be targeted on-line using any one or a combination of drug profile or demographic parameters and a range of appropriate services offered.

The level of complexity of these services ranges from promotional activity e.g. marketing diabetic jams to patients using insulin or reminders for all OAPs to get annual anti-flu vaccination to support activity for new therapies. The provision of this link into various patient groups' homes can be used to provide other information (health related or otherwise) concerning services or products which may be of some use such groups of patients.

In the latter case, a Drug manufacturer can obtain a list of codes of all new users of their products and send them support messages via the system. These may include, from day to day, electronic questionnaires about product usage. The response information can be passed back to the prescriber (GP) by the patient's pharmacist (who knows their name) in time for a follow up consultation. This feedback information is very important in correcting the misuse of drugs and providing targeted help to patients.

The drug company can use this feedback service as part of the process of persuading the GP to prescribe their product in the first place and this is therefore a very important marketing tool particularly in the launch of new products.

For the GP, the introduction of this system means that they now have additional information on which to deliver enhanced outcomes through patient focused healthcare. Also it can be seen that the system enables the trusted pharmacist moves to centre stage as a vital part of the team bringing valuable new services to patients to keep them healthy.

Patients complying with their drug regimes not only bolsters pharmaceutical companies (already high) profits, but also often reduces costs later in secondary healthcare. Not least it also often means better patient outcomes and lifestyles.

Other targeted messages from the drug companies, health associations, pharmacies, news agencies and the government help patients in understanding their illnesses and adjusting their lifestyles. This can include picture, audio and film and on-line interaction.

This level of support is currently unavailable and the system according to this aspect of the present invention is designed specifically to enable it.

A combination of a patient information management system described above and a pharmacy processing means connectable via a communications network to the communications means for receiving and processing the request from the system and notifying the system when the prescription is ready for supply. This enables pharmacies to be connected to the system to enable the creation of PDRs and to transmit that information to the system such that improved healthcare for the patient can be managed centrally. This is particularly useful when there are many pharmacies which the patient uses because all of the information from those pharmacies can be collated.

The pharmacy processing means may comprise a database of patient drugs records each having a unique identifier corresponding to the unique identifier stored in the database of patient records in the system. This enables the patient to have unique access to their records without the patient's personal details being available in the system. The confidentiality of the patients data records is maintained whilst enabling better services to be provided in support of the desired better management of patients.

The pharmacy processing means may comprise means for receiving a repeat prescription request and stock level means arranged to considering a stock list to determine the availability of a stock item specified in the request, wherein the stock level means is arranged to place an order for the stock item if, after meeting the request, the amount of the stock item will fall below a predetermined level. The ability to order stock immediately such that when the actual repeat prescription is received from the GP, for example, speeds up the delivery of the prescribed items to the patient because any delay due to ordering out-of-stock items is minimised.

It is also possible to collate information on the operation of pharmacies connected to the system and so the potentially lucrative data of the dispensing activities of the pharmacy can be obtained. The recent ruling on confidentiality of prescription information allows pharmacies to obtain an income from the sale of their computer data. Pharmaceutical companies value this information extremely highly.

The first aspect of the present invention also extends to a method of enhancing a patient's viewing of their personal patient drugs record, the method comprising: creating a patient drugs record at a pharmacy, the record providing personal information about the patient, which does not reveal patient identity, and being identifiable by a unique pharmacy-assigned identifier; transmitting the patient drugs record to a central site; receiving and storing the patient drugs record at the central site; storing support information at the central site received from a support organisation, the support information comprising matching requirement data associated therewith for matching the support information with a specific category of patients; and matching the requirement data with the personal patient information such that any patient access to the drugs record enables provision of targeted support information to the patient.

According to a second aspect of the present invention there is provided a prescription reordering system for requesting remote repeat prescriptions for patients, the system comprising: communications means arranged to receive a request for a repeat prescription via a data network, to forward the request to a patient-specified pharmacy, to receive a corresponding response regarding the allowance of the request and to store the response for patient consideration; and status indication means arranged to be updated by the patient-specified pharmacy to indicate to the patient the current status of the repeat prescription process and to specify when the prescription is ready for delivery or collection.

By the patient knowing when the prescription is ready, the prescribed medicine can be made available quickly without undue delay. The pharmacist does not have to chase the patient for them to pick up the medicine and the patient does not need to keep visiting the pharmacy to check whether the prescription is ready. Rather, the patient can arrange a convenient time to collect or receive the prescription, safe in the knowledge that the prescription is actually ready.

This decoupling of the patient from the pharmacist is also advantageous in that it frees up the pharmacist to carry out more of their prescription preparation work rather than answer patients' enquiries by telephone or in person, as to whether their prescription is ready.

Furthermore, the system embodying this second present aspect of the present invention enables the patient to determine from the status indication means which part of the repeat prescription process was taking the most time. Accordingly, if a patient has a particularly slow GP or pharmacist, they would be able to see this in the time it takes for each stage of the repeat prescription process to be carried out. If desired they could then use this information to determine whether they wished to change their pharmacist or GP. This transparency of the repeat prescription process leads to competition in repeat prescription handling which only improves the service provided to patients.

The second aspect of the present invention also extends to a method of requesting remote repeat prescriptions for patients, the method comprising: receiving a request for a repeat prescription via a data network; forwarding the request to a patient-specified pharmacy; receiving a corresponding response regarding the allowance of the request; storing the response for patient consideration; and updating status indication means with information supplied by the patient-specified pharmacy to indicate the current status of the repeat prescription process and to specify when the prescription is ready for delivery or collection.

According to a third aspect of the present invention there is provided a patient information management system for enhancing checking of a patient's personal patient drugs record, the system comprising: communications means arranged to receive patient drugs records created at pharmacies connectable to the system via communications links; a database of the received patient drugs records, each record uniquely identifying the issuing pharmacy and providing personal information about the patient including a list of the drugs prescribed by that pharmacy to that patient; means for enabling the patient to specify all of the pharmacies which they use; means for linking all of the pharmacy-specific patient drugs records of that patient together; and a drugs interaction assessment engine for considering the interaction between any of the drugs listed in the linked patient drugs records.

Currently each pharmacy has its own database or collection of PDRs which it has generated and may, if the facilities are available, carry out checks for drugs interactions using the PDR of the patient. However, if the patient uses another pharmacy for another prescription, because for example they do not want one pharmacist to know about a particular medication they are taking, the drugs interaction check is unable to cross check against all of their medications for interactions thereby limiting its usefulness.

The system according the third aspect of the present invention overcomes the above described problem of carrying out drugs interaction checks for patients who have PDRs at different pharmacies. As has been mentioned, PDRs are pharmacy specific as they are created by each different pharmacy. However, the system according to the present aspect of the invention links all PDRs together such that correct interaction checks can be carried out in consideration of all of the patients medications. Because each PDR used in this check is anonymous, the patient's confidentiality is not compromised in any way. Also the dispensing pharmacy where a new item is to be purchased can be made aware of the results of possible drug interactions. The receiving pharmacy is told about the possible interaction but is not told why, namely the contents of the patient's other PDRs at other pharmacies are still kept confidential.

Preferably, each PDR has a unique pharmacy-assigned identifier and the personal information about the patient does not reveal patient identity. The anonymity of the PDR provides further assurance to the patient that their PDR remains confidential at all times as unauthorised access to the PDRs at the system site would still not reveal the patient's identity.

The system may further comprise a database of patient demographic data records, each record providing details regarding the patient but not revealing the patient identity, namely not providing the patient's name or address. This enables the system to have more information than just the patient's PDR available such that patient interaction with the system can be enhanced. For example, patients can carry out their own drug interaction tests by logging on to the system, proving their identity by passing a security check (e.g. a password check), and then entering the name of an over the counter (OTC) drug which they wish to purchase. The system could then carry out a drug interaction check on all of their PDRs and advise on the suitability of the OTC drug without troubling the pharmacist for this information or indeed with the patient having to visit the pharmacist themselves.

The third aspect of the present invention also extends to a method of enhancing checking of a patient's personal patient drugs record, the method comprising: receiving patient drugs records created at pharmacies connectable to the system via communications links; storing the received patient drugs records, each record uniquely identifying the issuing pharmacy and providing personal information about the patient including a list of the drugs prescribed by that pharmacy to that patient; enabling the patient to specify all of the pharmacies which they use; linking all of the pharmacy-specific patient drugs records of that patient together; and considering the interaction between any of the drugs listed in the linked patient drugs records.

Patients registering to a system (described in detail later) embodying the above-described aspects of the present invention at their pharmacy or pharmacies have on-line access to their dispensing record (PDR) held at that pharmacy. The system allows them to place repeat prescription requests on their GP surgery via their pharmacy and to monitor the status of the request. Messages between patients and their GPs are also supported by the system and provide a convenient way of patient-doctor communication. These messages cannot be edited by the pharmacy as the request is either made directly from the patient or from the GP.

Status request tracking is performed as an automatic updating of the Web site as the pharmacy carries out the request processes. When the message is sent, the Web site shows a tick in the box that indicates that the request is at the pharmacy. When the pharmacy prints out the request to take to the surgery or otherwise sends it on, then the Web site moves the tick along to the box indicating that the request is at the surgery. When the signed prescription form arrives at the pharmacy from the surgery the pharmacist clears it from the request file on his system which automatically updates the Web site moving the tick along to the box indicating that the prescription is either ready for delivery or collection.

This prescription request process provides a rationale for patients to access the system (Web site) at least once in every twenty-eight day cycle and probably two to three times within this period. This provides a realistic basis for offering patients entirely new on-line services to support the treatment for their health condition.

For security reasons, system does not store patient names and addresses at all (neither within its drug records or its patient records) although other patient demographics including age and sex are kept. PDRs are known to the system by the PDR number which was issued to them by their pharmacy having been firstly allocated to that pharmacy by the system. All communications to patients are via the system, effectively creating a patient/pharmacy intranet. All messages from subscribing organisations are submitted in predefined format which has been scrutinised and approved by the system. New messages for patients from existing subscribers will not be accepted unless they conform to one of the predefined formats as determined by a filter engine. This security protocol ensures that the system is able to filter all communications to individual patients thus protecting them from undesirable, potentially harmful or inappropriate marketing.

Drug companies can also use the system to provide updated information on product range, OTC medicines for minor ailments that are compatible with patient drug profile and lifestyle/dietary implications with recommendations.

At the system Web site patients can check whether prospective OTC product purchases are safe (interaction free) to take with their prescription medicines. This is achieved by either typing the name of the product or typing the name of the therapy group (e.g. tickly cough) for a list of all safe products. Interaction checks are run against a consolidated drug record made up of all of the patient's drug records from multiple pharmacies. The consolidated drug record is created by the patient entering all of the unique PDR numbers which have been acquired by the use of different pharmacies. All of the records associated with these numbers are collated/linked by the system such that the patient has a network of pharmacies which can be used with the consequential advantageous automatic updating of the centrally held PDRs.

This is a unique and valuable service. At present pharmacies can only inform patients of interactions with drugs that have been dispensed at that pharmacy since records are only held locally. This information can be inaccurate by exception and therefore potentially dangerous to patients.

The system allows pharmacies access to all their patients data (via pass key codes, also available to the patient's GP) so that they can view or enhance the information patients are receiving and be prepared to answer on-line any questions they may have.

For the pharmacist the system adds an entirely new dimension to patient interaction especially with permanently or temporarily housebound patients. It prepares the way for introduction of on-line medicine management services and repeat prescribing within the pharmacy.

The system allows better stock control procedures to be introduced since the pharmacy is aware in advance of upcoming prescriptions.

The system of the present embodiment allows control of pharmacy chains from anywhere in the world. Managers can extract pharmacy data in report format (based on data generated by each of the designated pharmacies) from all pharmacies in the group from any internet access point and can communicate appropriately with the pharmacies. This gives managers unprecedented up to the moment information on pharmacy performance using real-time data which provides a visual appreciation of current situations.

It is possible for registration of an institution (residential home/prison) as an entity with the residents as sub-entities. This enables more efficient support for the institution such as a nursing home and its patients, as it cuts down the amount of paperwork required to obtain the medication for the new or repeat prescriptions.

The present embodiment has a Web-linked stock control system. Patient-generated product requests are automatically checked against the pharmacy stock position and debited accordingly in anticipation of an imminent prescription. Also any items noting stock can be instantaneously ordered by simply sending an e-mail to the relevant supplier. This has the advantage of product being in stock when the patient prescription arrives, removing the need to owe items or disappoint the patient entirely. The pharmacy is also better able to monitor stock and manage cash flow.

According to a fourth aspect of the present invention there is provided A graphical user interface (GUI) for use by pharmacists in processing prescriptions, the interface comprising: a prescribed drugs data selection portion arranged to enable the pharmacist to compose an on-screen representation of the prescription information presented on the prescription; means for generating an on-screen graphical representation of a government-required endorsement portion of a prescription, the generating means being arranged to convert the information selected in the prescribed drugs data selection portion or information related thereto into a format required for completion of an endorsement section of a prescription; and an editing tool for editing the contents of the endorsement portion prior to finalising its content.

Provision on screen of this information enables the pharmacist to see the endorsement data before it is finalised and this enables it to be edited if incorrect. In other prior art systems, either DOS or Windows programs, the visual display is not available and medicines are dispensed one at a time. The pharmacist is not able to check the "endorsement" until completion. So if it is incorrect then something else has to be done or the prescription has to be manually amended. The present invention also enables the user to change the order in which the items are listed for printing in the endorsement section of the prescription. This advantageously prevents the problem of the prescribed items not matching up with the endorsement data.

The generating means is preferably arranged to generate pack data for specifying the actual quantity of the drug dispensed in packs to meet the prescribed quantity of the drug.

The GUI may further comprise a portion for displaying the personal details of the patient and also a portion for displaying the details of the prescribing person, for example the patient's general practitioner. This provides further information for use with the patient's drug record and also meets the government regulations for account of prescribed drugs.

The data entry portion and the endorsement portion may be arranged to simulate a government accepted prescription endorsement form. The appearance of this on screen provides the pharmacist with a familiar format which is readily understandable.

Preferably, the GUI further comprises means for creating a dispensing label for the prescribed medicine from the entered prescription information and a label imaging portion for viewing a simulation of the printed label prior to its being printed. Clearly, this also helps avoid any errors in data entry as the pharmacist is very aware of the significance of errors in the labelling of dispensed drugs.

The GUI may further comprise means for entering patient identifying information, means for linking the current prescription to a patient drugs record stored at the pharmacy and a patient drugs record displaying portion for displaying the stored patient drugs record. Again this additional information provides a better all round picture of the patient and thereby assists the pharmacist in detecting errors and inconsistencies which may be in the GPs prescription.

The GUI may also comprise means for indicating the time duration since each of the drugs in the patient drugs record was dispensed.

Furthermore the GUI may comprise means checking the entered prescription information against known combinations of drug interactions and for displaying the results to the pharmacist.

### Brief Description of the Drawings

In the drawings:
Figure 1 is a schematic system diagram showing a distributed patient information management system embodying the present invention, the system including an Enigma processing centre and at least one pharmacy server;
Figure 2 is a schematic block diagram showing the Enigma processing centre of the patient information management system of Figure 1 in detail;
Figure 3 is a schematic block diagram showing a pharmacy server of the patient information management system of Figure 1 in detail;
Figure 4 is a schematic block diagram showing the databases stored at the Enigma processing centre of Figure 2;
Figure 5 is a schematic block diagram showing the databases stored at the pharmacy server of the patient information management system of Figure 3;
Figure 6 is a schematic block diagram showing the structure of a patient record in the patients databases of Figure 3 and 4 at the Enigma processing centre and at the pharmacy respectively;
Figure 7 is a schematic block diagram showing the structure of a patient drugs record in the patients drug databases of Figure 3 and 4 at the Enigma processing centre and at the pharmacy respectively;
Figure 8 is a flow diagram showing a method of a patient registering with the distributed patient information management system of Figure 1;
Figure 9 is a schematic block diagram showing the configuration of the Web site at the Enigma processing centre;
Figure 10 is a flow diagram showing a method of process of ordering a patient obtaining a repeat drugs prescription using the distributed patient information management system of Figure 1;
Figure 11 is a schematic diagram of a Enigma processing centre Web site page showing the status of repeat prescription process shown in Figure 10; and
Figure 12 is a screen dump of a Graphical User Interface provided at the pharmacy server of Figure 1 for entering patient prescription data.

### Detailed Description of a Presently Preferred Embodiment of the Present Invention

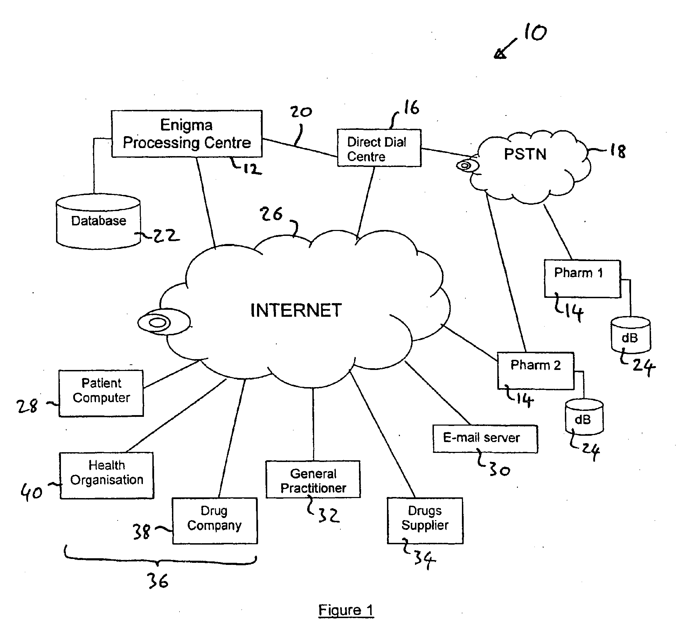

A distributed patient information management system embodying the present invention is now described with reference to Figure 1. The system 10 essentially comprises an Enigma processing centre 12 and several pharmacy servers 14, each server 14 being provided at a respective pharmacy premises. In this embodiment, only two pharmacy servers 14 are shown though in practice there may be hundreds. Each of the pharmacy servers 14 is connected to the Enigma processing centre 12 via a direct dial centre. More specifically, each pharmacy server 14 connects to the direct dial centre 16 through a Public Switched Telephone Network 18 and the direct dial centre 16 is connected though a special leased line telecommunications channel 20 to the Enigma processing centre 12. This connection forms the main communication channel between the Enigma processing centre 12 and the pharmacy servers 14.

The Enigma processing centre 12 has a local SQL database 22 which itself accommodates a plurality of smaller more specific databases that are described later. Similarly, each pharmacy server 14 has its own local database 24 which stores a plurality of locally used databases that are also described later.

The Enigma processing centre 12, the direct dial centre 16 and the Pharm 2 pharmacy server 14 are each connected to the Internet 26 to enable wide area network communications. Even though the Pharm 1 pharmacy server 14 does not have a direct link to the Internet 26, it can be linked to it via the direct dial centre 16. The connection of the pharmacies 14 and the Enigma processing centre 12 to the Internet is important as it enables patient interaction from a patient's computer 28 connected to the Internet 26. Also as an e-mail server 30 is also connected to the Internet 26, e-mail messages can be sent to a general practitioner (GP) 32 of the patient. Similarly, the Pharm 2 pharmacy server 14 can send messages in a very simple manner to its drugs supplier 34 via the e-mail server 30. Finally support information suppliers 36, such as a drugs company 38 and a health organisation 40, can send their support data to the Enigma processing centre 12 via the e-mail server as an e-mail message or can download the support data to the Enigma processing centre 12 Web site directly.

The Enigma processing centre 12, acts as a central hub for all patient enquiries. Its main purpose is to provide a means for a registered patient to view remotely and securely its patient history in the form of the Patient Drugs Record (PDR). However, it is also able to facilitate requests for repeat prescriptions, to monitor the status of those requests, to provide targeted support information to the patient, to warn against possible drug interactions even when the patient uses more than one pharmacy for their prescriptions, and to identify patient groups, e.g. patients of a nursing/residential home, patients of a particular GP or practice or patients with a particular chronic illness etc.

The pharmacy servers 14, create PDRs, provide stock management and an improved re-order process of drugs with suppliers, handle repeat prescription requests, control the status of such requests, handle Monitored Dosage Systems (MDS) involving prescription requests from patients groups, deliver messaging between GPs and patients and between drugs companies and GPs, check for drug interactions and provide improved pharmacist interaction with prescription handing processes by way of an improved Graphical User Interface (GUI).

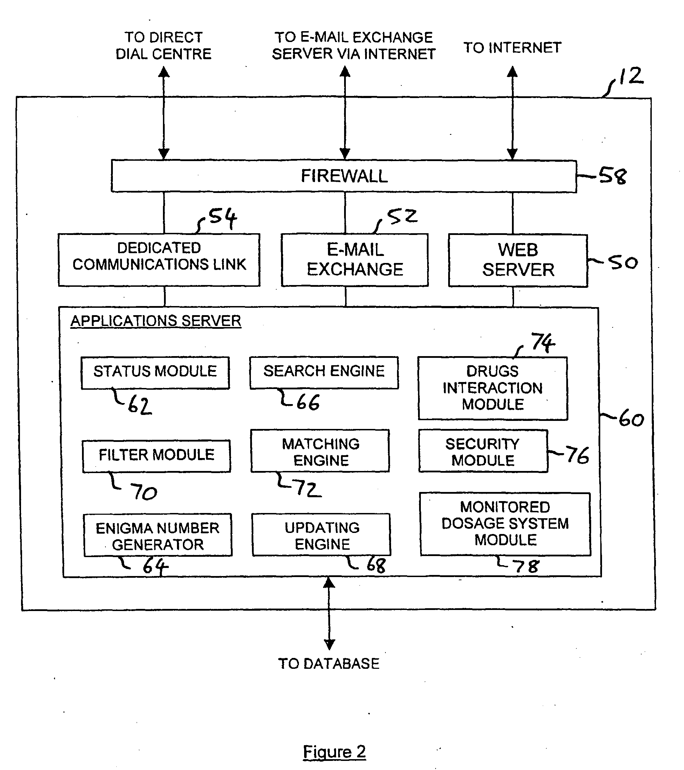

Referring now to Figure 2, the Enigma processing centre 12 is made up of a plurality of processing units. More specifically it comprises a Web server 50 for handling communications to and from the Internet 26 and hosting the Enigma Web site, an e-mail exchange module 52 for receiving and sending e-mails to the e-mail server 30, and a dedicated communications link module 54 for connecting the processing centre 12 to the direct dial centre 16. All of the external communications to and from the Web server 50, the e-mail exchange module and the communications link module 54 are routed though a firewall 58 to provide a secure and safe way of handling external communications. The firewall 58 ensures that any unrecognised format of communications are filtered and can, in the case of the dedicated communications link module 54, ensure that communications are only to and from the direct dial centre 16 via the telecommunications channel 20.

The Enigma Web site hosted by the web server 50 is designed to provide services to Enigma registered patients, pharmacies and GPs. While any one on the Internet 26 may access the site, no services other than the processing centre's public domain information is available to the persons looking at the site. They must have a valid Enigma number (PDR number) to get access to any of the Enigma's repositories of information as will be described later.

Internal communications from the Web server 50, the e-mail exchange module 52 and the dedicated communications link module 54 are with an applications server 60. This in turn can send and retrieve data from the local SQL database 22, acting as a central repository, and all of the smaller databases contained therein.

The applications server 60 is comprised of several applications modules and processing engines. The server 60 includes a status module 62 for monitoring the status of a patient's request for a repeat prescription. The status module 62 is responsive to status updates from the pharmacy server 14 to provide a readily comprehensible display to the patient for indicating how far down the repeat prescription process the request has reached and ultimately to indicate when the prescription is ready for collection or delivery.

An Enigma number generator 64 is also provided in the applications server 60. The generator 64 generates PDR numbers (Enigma numbers) and assigns batches of the PDR numbers to each registered pharmacy server 14, for example PDR numbers 4AAA000 to 4AAA999 for Pharm 2. These numbers are used by the pharmacy when a new patient record is created at the pharmacy, with each new patient record having a unique unused PDR number. At no point can anyone create or assign a PDR number manually, the generator 64 and the pharmacy server 14 deal with this automatically.

The application server 60 includes a search engine 66 which enables a patient to search for and locate a given pharmacy via any of its stored fields including its name, location, and a PDR number. This is an important facility when the patient uses several pharmacies and cannot remember which pharmacy he used for the prescription or when a patient is looking for a local pharmacy which has registered with the Enigma processing centre 12.

A registered pharmacy server 14 can upload its information to the database 22 of the Enigma processing centre 12 and in order to do this an updating engine 68 is provided in the application server 60. The updating engine 68 ensures correct updating of the appropriate records stored in the appropriate database within the local SQL database 22.

The applications server 60 is provided with a filter module 70 and a matching engine 72. The filter module 70 considers the support information sent to the Enigma processing centre 12 by the support information suppliers 36 and removes all information not suitable for passing onto the registered patients. Some of the ways in which the information is filtered is by considering whether the support information meets a predetermined acceptable format and by considering from whom the support information is received. In the present embodiment, the support information is provided with matching requirement data specifying parameters for matching the support information with a specific category of patients, for example a demographic requirement can be "all patients over the age of 65" and a prescription related requirement may be the types of drugs being prescribed. The parameters of the matching requirement data can also be combined to make the targeting more specific. Thus the support information is targeted at specific patients. These may be either relevant to patient's drugs or medical condition.

The matching engine 72 is responsible for using the matching requirement data as search criteria to find stored PDRs in the database 22 which have fields meeting the criteria. These PDRs are then considered to be matched to the support information. The matching engine 72 then links the support information to the matched stored PDRs such that when a patient logs on and accesses their PDR, they are also presented with the supporting information.

The applications server 60 also comprises a drugs interaction module 74, a security module 76 and a monitored dosage system (MDS) module 78. Each of these are now described briefly.

The drugs interaction module 74 is arranged to check the interaction between a new drug and the drugs in the PDR, for example when the patient purchases an over the counter (OTC) drug and wishes to know if it is safe to use with their prescription drugs. The drugs interaction module compares the drugs against a stored database of dangerous combinations of drugs and can inform a patient who has for example made an inquiry though the Web site, whether the OTC drug is safe to use.

Interaction checks are run against a consolidated drug record made up of all of the patient's drug records from multiple pharmacies. The consolidated drug record has previously been created by the patient entering all of the unique PDR numbers which have been acquired by the use of different pharmacies. All of the PDRs associated with these numbers are linked by the drugs interaction module 74 such that the patient has a network of pharmacies which can be used with the consequential advantageous automatic updating of the centrally held PDRs.

The security module 76 deals with secure patient access to the database 22 where sensitive PDRs are stored. The security module 76 runs password and user name security protocols to ensure that only genuine registered patients can have access to their PDRs. The security module 76 also encrypts and decrypts messages sent between the Enigma processing centre 12 and the direct dial centre 16, the pharmacy servers 14 or the GP 32. Other standard encryption security measures can also be handled by the security module 76.

The MDS module 78 is used for patients who are registered at nursing homes and have to be given drugs at certain time intervals. The MDS module 78 handles the creation and usage of Medicine Administration (MAR) charts which enable the ordering and dispensing of drugs prior to a prescription having been created for the required drugs. The MDS module 76 allows a representative of the nursing home to construct a group of patients for whom the MAR charts are to be created and sent in a request for the drugs. Also for each patient, the MDS module 78 enables the representative to select the medicines that are to be administered (name, strength, form, quantity), enter the dosage regime and enter the number of times that each item is to be administered. Also the date range for which the MAR charts are applicable can be specified as can the dosage dispensing system. There is also a facility to view the old MAR charts if required.

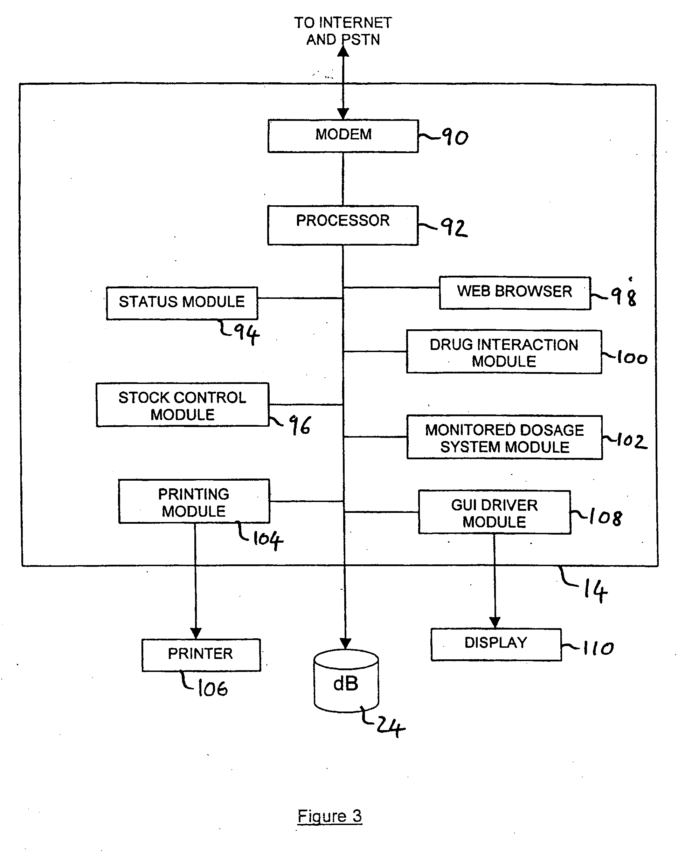

Referring now to Figure 3, the Pharm 2 pharmacy server 14 is connectable to the Internet 26 via a dial up modem 90. The pharmacy server 14 comprises a central processor 92, namely that of a personal computer, and various software modules running on that personal computer. The software modules include a status module 94 for monitoring the various stages of processing of a received repeat prescription or MAR chart request and for communicating the status back to the processing centre 12, a stock control module 96 for monitoring the stock levels stored in the database and for ordering further stock from the drugs supplier 34 when they fall below a predetermined amount, a web browser 98 for Internet navigation, a drugs interaction module 100 for carrying out local drug interaction checks, and an MDS module 102 for handling MAR chart requests.

The pharmacy server 14 also includes a printing module 104 which controls the printing at a printer 106 of specific labels for dispensed drugs according to the prescription. The printing module 104 can co-operate closely with other modules such as the MDS module 102 when labels are required in various specific formats to facilitate the nursing staff in administration so that patients get the medicines correctly.

A GUI driver 108 is also provided for generating an interface including an OSCAR on a computer display 110 of the pharmacy server 14. The GUI driver 108 controls a pharmacist's interaction with the pharmacy server 14 and assists in dispensing of the drugs. This interface and the OSCAR is described in greater detail later.

Referring the MDS module 102 in more detail, it is arranged to create the OSCAR from a MAR chart request for dispensing to take place in the same way as for any other NHS prescription. However, one specific feature of a MAR chart is accommodated, where more than one medicine is present in a single compartment (of a daily or weekly tablet dispensing box), the MDS module 102 has a facility for tablet and capsule identification to be made in terms of colour, shape and markings. Control of the printing module enables various MAR charts formats, backing sheets and repeat forms in different formats to be printed. When dispensing drugs to the nursing home patients, the MDS module 102 automatically updates the MAR Chart, and the pharmacist is presented with a separate screen, which is specific to the nursing home.

The MDS module 102 is able to produce various reports, which can be sent to the nursing homes along with the drugs. (for example a picking list, a patients list, dosage instructions for each drug patient wise etc.) The ordering of these reports is either on patient name or by the room number in the home.

Normally the prescriptions for such patients are received from the GP some time after the drugs have been dispensed to the homes. Accordingly a facility is provided in the MDS module 102 for endorsing these prescriptions after verification and reorder of items on the prescription.

The stock control module 96 is configured to have the following stock control features:
- an ability to request ordering of an item, or items, during or at the end of the dispensing process, for items that have just been dispensed or are owing;
- an ability to add items to an order in an ad-hoc manner, especially for OTC products that do not require the production of a label;
- a facility which alerts the pharmacist that a quantity of an item is already on the current order list;
- a facility that allows the pharmacist to select the wholesaler (supplier 34) to which the order is to be sent;
- an automated facility to link to the supplier 34 via the modem 90. This facility will not cause the loss of any activity being carried out by the pharmacist at the time of connection and communication, i.e. products are ordered by background transmission of a request to the supplier 34;
- a facility to display details about the order transmitted by a supplier's computer system 34, and an ability to respond (e.g. supplier's system 34 responds to an order by stating that it is out of stock of a particular item and asking to confirm or cancel);
- an ability to order on a one for one basis i.e. use one, order one;
- an ability to print orders for faxing to the supplier 34;
- an ability to amend the order quantity calculated by the stock control module 96 before placing an order;
- an ability to place items on different order pages. When a drug is dispensed for the first time the default is to place it on a general order page. The pharmacist may select to move such items to different order pages. Upon subsequent dispensing of the drug, the stock control module automatically places it in the respective order pages;
- an ability to see the Maximum Daily Usage (MDU) per item per day;
- a provision to store three months MDU and eleven months MDU upon a pharmacist's request;
- an ability to calculate the order quantity on the basis of the following:
   Minimum order quantity
   Maximum order quantity
   Number of days in stock
   MDU
   Re-order level
   Current stock
   Order outstanding

The stock control module 96 has a semi-automatic ordering feature which works on the following basis. The pharmacist enters the quantity and usage into the stock control module 96 when he/she places an order a product for first time. If not, the stock control module 96 places it on the order page showing a zero quantity with the usage in parentheses. However, as soon as the pharmacist orders the product for the first time, the stock control module 96 works out a semi-automatic ordering request as explained below:

| **Example of Usage & Ordering:** | | | | |
|---|---|---|---|---|
| **Example No.** | **Example Description** | **Quantity** | **Pack Size** | **Usage** |
| (1) | 30 Tabs dispensed out of a bottle of 500 Tablets | 0 | 500 | 30 |
| (2) | 3 x 15g Special Containers dispensed | 3 x 15g | 15g | 45g |
| (3) | If 198 tablets dispensed out of mixed packs (6 0 x 28 & 30 out of 100 pack) | 6 x 28 0 | 28 100 | 168 30 |

Case (1) above shows zero quantity on the supplier page, because the pharmacist has not ordered the product. Case (2) shows a simple replacement system. Case (3) above is a combination of full packs and a part quantity being dispensed from a full pack.

The pharmacist can edit the quantity (order) field and once an order has been transmitted the usage figure on screen becomes the usage since the last time ordered. Also, once an order has been transmitted, all products with a quantity in quantity field are treated as items received and do not appear again. But, the products with zero quantity in quantity field disappear this time until used again to trigger an order.

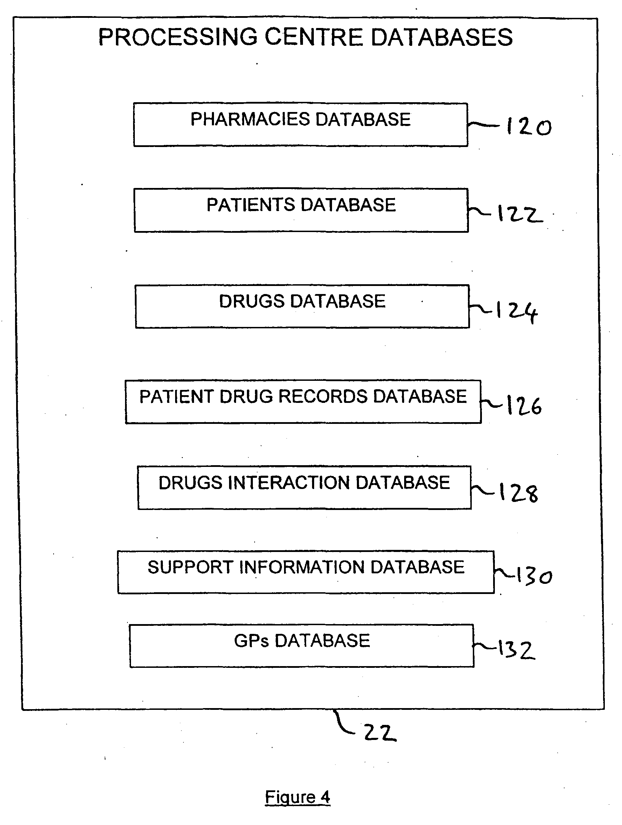

Referring now to Figure 4, the Enigma processing server database 22 comprises a plurality of specific databases. More particularly, the database 22 includes a pharmacies database 120 listing details of all registered pharmacies, a patients database 122 providing personal details of each patient, a drugs database 124 setting out the different types of drugs available and the usage of those drugs, a PDRs database 126 supplied from the pharmacies, a drugs interaction database 128, and a support information database 130 storing information to be targeted at specific groups of patients. Any changes to PDRs are also communicated to the pharmacies storing information relating to that PDR for the update of their local databases 24.

Finally a GPs database 132 is provided which includes details of all NHS registered GPs 32. This list is kept up-to-date and is downloaded to each registered pharmacy server 14 to update their records.

The pharmacies database 120 contains a set of pharmacy records (not shown) describing each of the pharmacies registered with the information management system 10. Each pharmacy is identified by its name, postal address, web address (optional) and telephone number, and for the purposes of linking other records to that pharmacy record, a unique pharmacy code is provided. Other relevant information about the pharmacy is provided such as the opening and closing times, the services offered and the pharmacy's PPA number.

Pharmacies may change their stored details locally at the pharmacy server 14, and then update their pharmacy record stored in the pharmacies database 120 at the Enigma processing centre 12.

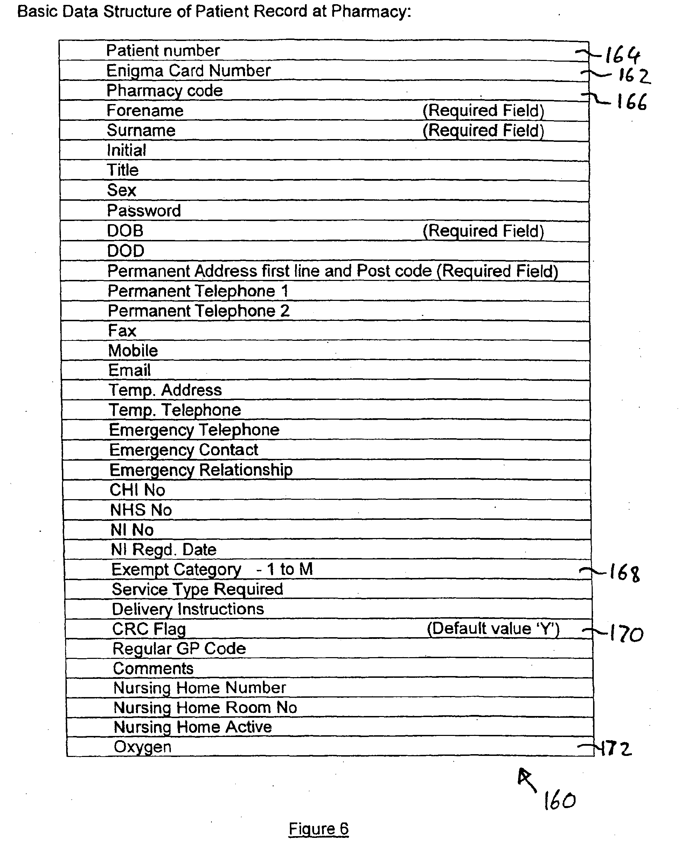

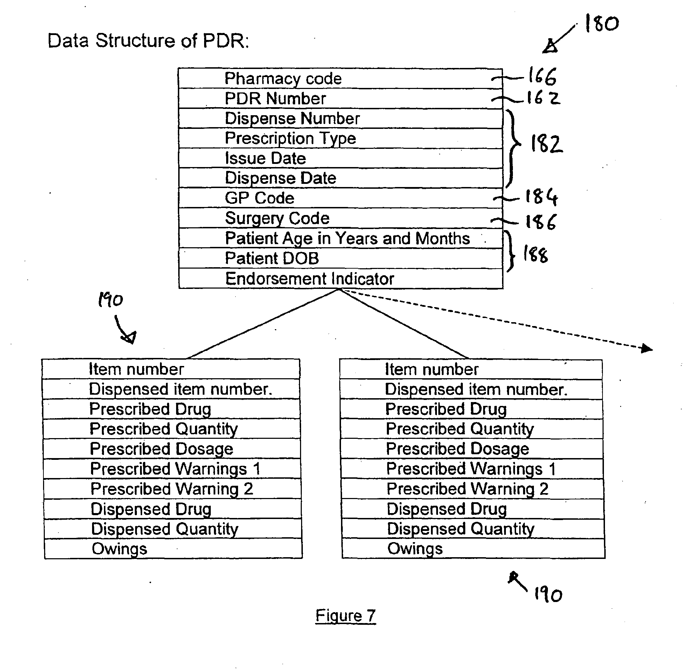

The patients database 122 comprises a plurality of patient records which are described later with reference to Figure 6. Similarly, the PDRs database 126 comprises many PDRs, the structure of which is described in detail later with reference to Figure 7.

The support information database 130 comprises different types of information depending on the supplier of the support information. For example, if the information is provided by the health organisation 40, it may include counselling information targeted at patients using antidepressants. Similarly, if the support information supplier 36 is a drugs company 38 it may include a questionnaire regarding the use of a newly prescribed drug. Feedback from the questionnaire can be forwarded onto the GP 32 such that the GP gets a better idea of how the prescribed drug is working.

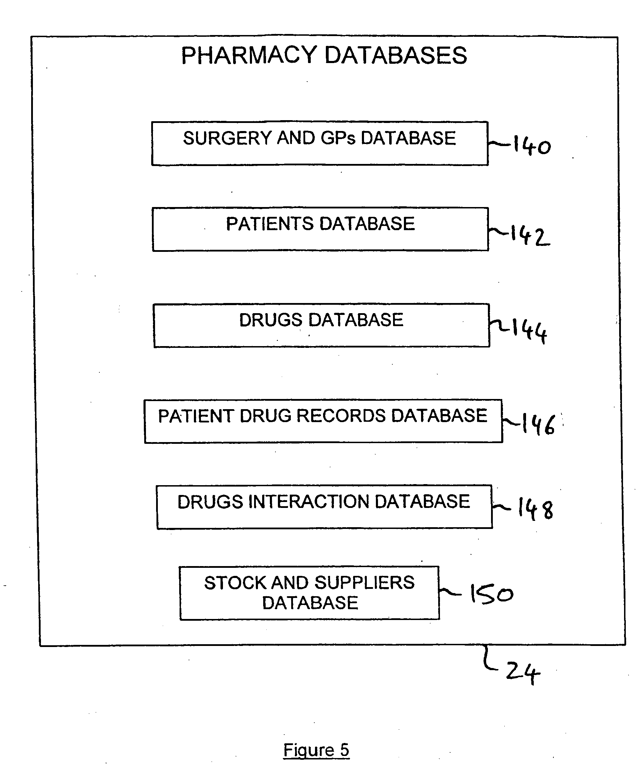

The pharmacy server database 24, as shown in Figure 5, also maintains local databases. A database 140 of records describing each patient's surgery and GP 32 is provided. The pharmacy server database 24 also comprises a patients database 142, a drugs database 144, a PDRs database 146 and a drugs interaction database 148. Each of these latter four databases is a local equivalent of the corresponding databases 122, 124, 126, 128, provided in the Enigma processing centre database 22, described previously. However, the PDRs database 146 and patients database 142 are locally relevant subsets of their counterpart databases 126, 122 in the processing centre database 22.

The pharmacy server database 24 also comprises a stock and suppliers database 150 which specify the stock levels of each item sold by the pharmacy 14 and list the suppliers 34 used to provide the stock.

All GPs 32 registered with the NHS are listed in the GPs and Surgery database 140. For every new Enigma pharmacy 14, a consolidated list of GPs is supplied from the GPs database 132 at the processing centre 12 to form a local GP database 140. The pharmacist may update the list if required and any changes made are sent back to the processing centre 12 for updating the GPs database 132.

Whilst not shown in Figure 5, other related information is also stored in the pharmacy server database 24 which enables a pharmacist to dispense prescriptions and to endorse them for invoicing to the NHS.

Referring now to Figure 6, the basic data structure of a patient record 160 of the patient database 142 of the pharmacy server database 24 is now described.

The record 160 specifies the PDR number (Enigma Card Number) 162, which has been assigned to that patient. This number is by no means a unique identifier of the record as a patient can collect several different PDR numbers if he or she uses several different pharmacies. Accordingly, a unique patient record number 164 is also assigned to the record 160. Also the unique pharmacy identifier code 166 is provided for enabling the pharmacy associated with the generation of the PDRs is identified.

Personal details about the patient including name, address, date of birth and sex are also provided as are other fields which will be clear from consideration of Figure 6. However, an exemption field 168 is provided which specifies the category of payment exemption which if applicable the patient is categorised under. Also the CRC flag field 170 simply specifies whether a Child Resistant Container is required.

The patient record 160 also comprises an oxygen field 172, which specifies whether the patient requires oxygen. This field is important as the dispensing of prescriptions containing oxygen is dealt with separately from the dispensing of other prescriptions. The information management system 10 is able to record and identify those pharmacies that supply oxygen. Each time a pharmacist dispenses a prescription for oxygen and/or oxygen equipment, the details of the equipment are maintained as well as the details of delivery and collection details are recorded (especially the distance of the patient's home from the pharmacy). The oxygen register reflects the hardware upon which the Health Authority will pay the monthly rentals. No endorsement is necessary on the oxygen prescriptions.

Whilst not shown in the figures, the patient record of the patient database 122 of the processing centre database 22, is very similar to the above described patient record 160. However, in order to ensure anonymity for the patient, the fields relating to the identity of the patient are removed. More specifically, the fields relating to the patient/nursing home name, address, telephone number, e-mail, and fax number are removed.

The PDR 180, shown in Figure 7, is central to the present information management system 10 and most of the dispensing process flows through it. It consists of a complete dispensing history for a patient registered with the pharmacy issuing the PDR 180. Each patient has his or her own PDR 180 and the entire group of individual PDRs 180 together make up the pharmacy PDR database 146. All the pharmacy PDRs 180, consolidated at the processing centre 12, make up the important processing centre PDR database 126 from which the range of central processing centre services are provided. The definition of a PDR is therefore critical to the entire information management system 10.

The key feature of the PDR 180 is that it is to the layman anonymous because there is not patient identifying personal information provided in the PDR 180. Rather the PDR has fields for the unique PDR number 162 and the pharmacy code 166 which link the PDR back to the pharmacy server 14 which issued the PDR 180 and which stores the patient identifying details in the patients database 142.

The PDR 180 also specifies information regarding the dispensing of a prescription 182, the GP identifying code 184, the surgery identifying code 186 and personal demographic information 186 such as age and data of birth.

For every PDR 180 there are one or more drug detail information sections 190, one per prescribed item. Each drug detail information section 190 sets out the prescription details such as prescribed drug, quantity prescribed, dosage, warnings, dispensed drug and owings.

An owings facility is provided in the pharmacy server 14 to record the quantity of any medicine(s) still owed to the patient. The pharmacy server 14 is capable of producing a label for the correct quantity of medicine actually dispensed and an owing slip. The pharmacy server alerts the pharmacist with on-screen reminders (appropriately recorded in the PDR 180, and in an owings register) that a patient is owed a medication. When a patient returns to collect his/her outstanding medication, corresponding label is produced.

An example of what an owing register may contain is set out below:

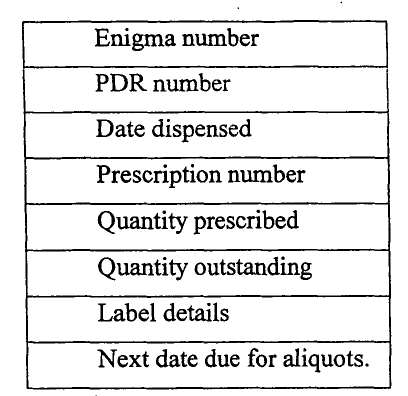

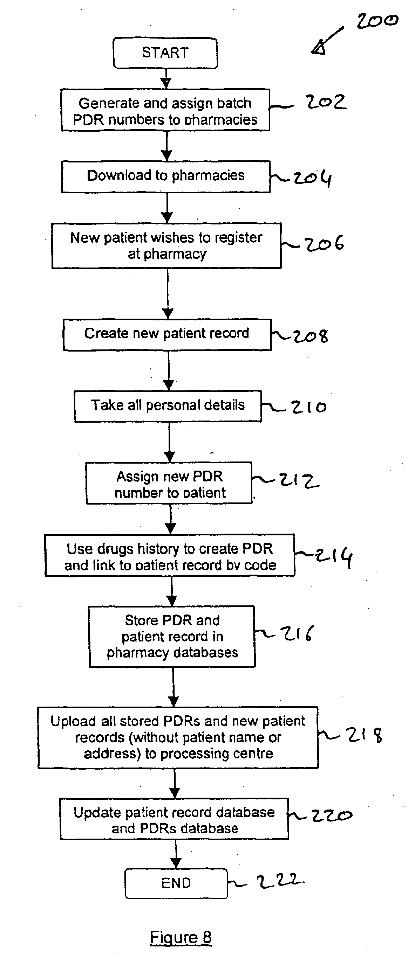

Referring to Figure 8, a method 200 of registering a patient with the information management system 10 is now described. The method 200 commences with the generation and assignment at Step 202 of batches of PDR numbers 162 to pharmacy servers 14. The numbers are generated in the Enigma number generator 64 of the processing centre's applications server 60. Then the batches of PDR numbers 162 are downloaded at Step 204 to the corresponding pharmacy servers 14.

New patients wishing to register with the information management system 10 can do so by going to the Pharmacy directly at Step 206 with a prescription. The pharmacy server 14 creates at Step 208 a unique patient record 160 and subsequently takes at Step 210 all of the patient's personal details for populating the patient record 160. Then one of the earlier pharmacy-assigned PDR numbers 162 is assigned at Step 212 to the patient and recorded in the patient record 160.

Next the pharmacist enters in at Step 214 all of the prescription data into the pharmacy server 14 and this information is then used to create the PDR 180. The PDR 180 is then stored at Step 216 in the PDR database 146. At the end of the day, all the patient records 160 (having had the patient's identification fields removed) and the PDRs 180 at the pharmacy server 14 are uploaded at Step 218 to the Enigma processing centre 12, where they are used to update at Step 220 the patients database 122 and the PDR database 126. This is then the end of the procedure at Step 222.

The pharmacy server 14 is able to produce and issue Enigma cards (with the PDR number 162 and the patient's name). These cards serve as a reminder to the patients where their PDR and patients records 160 are held. When the patient registers with the pharmacy of his/her choice for the first time, a label is printed for fixing on an Enigma card. Accordingly, the patient is also provided during the registration process 200 with an Enigma card containing their PDR number 162.

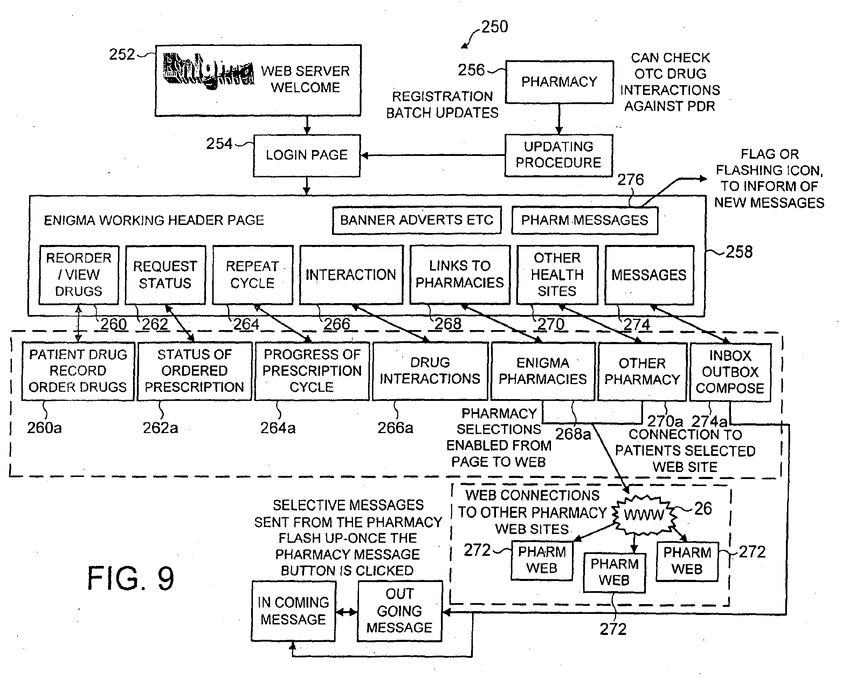

Referring to Figure 9, the configuration of the Enigma processing centre Web site 250 is now described. The main purpose of the Web site 250 is to enable patients to order their repeat prescriptions via their chosen pharmacies, to have complete access to their own personal drug records, as well as receive targeted support information such as that regarding, medical reference and counselling. In short, the Web site 250 provides the access to the Enigma processing centre 12 which enables it to act as a complete reference library for all patient subscribers. The information available to the patient subscribers may be specific or general in nature. Emphasis is placed on security of patient specific information, and information flow.

The Web site 250 comprises a welcome home page 252 followed shortly thereafter by a login page 254. The login page 254 requires the patient to enter its user name and password. In order to receive the required password, the patient has to have obtained the PDR number 162 from the pharmacy 256 and have completed the previously described registration procedure 200. Once the patient has received his or her PDR number 162 they can login and set their own passwords. Passwords may be changed regularly. These passwords are available in the Enigma processing centre 12 and if patients forget their passwords, they may be able to retrieve these from the pharmacy 256.

Following successful login, the patient is taken to the working header page 258. Here the options provided include reorder/view drugs 260 which enables the patient to view 260a their own complete drug record from the centre and order 260a their repeat drugs from the specified pharmacy, including collection of prescription and home delivery as appropriate. A request status and repeat cycle options 262, 264 respectively, enable the patient to view 262a the status of their ordered prescription (ready for collection or delivery) and to monitor 264a the progress of the prescription cycle (at pharmacist, at GPs).

An interaction option 266 provided for checking 266a if there is any drug interaction of an OTC with patient's own PDR 180. Options are provided for links to pharmacies 268 which direct the patient to Enigma registered pharmacies 268a and links to other health Web sites 270 which lead the patient to other pharmacies 270a, for example. With either of these selections, the patient can be led to pharmacy Web sites 272 via the Internet 26. This enables patients to access a registered pharmacy's own Web site where available, to select their preferred pharmacy, and to access their preferred pharmacy.

The other patient selectable option from the working header page 258 is that for sending/receiving/composing messages 274 usually to pharmacies. Here messages can be placed in an outbox 274a, accessed from in inbox 274a or just created in a text composer 274a. As per standard e-mail procedures messages in the outbox are delivered and incoming messages are received and out going messages are delivered at appropriate times.

The working header page also comprises a pharmacy message flashing icon 276 to alert the patient of an incoming message from the pharmacy. This may be an update in the status of a prescription cycle for example or a selective message to assist in patient care.

Some further rules and facilities of the Web site 250 are set out below. Patients are only able to examine their own PDRs and not anyone else's for obvious security reasons. Where a pharmacy has its own Web site, a facility is available where the patient is automatically taken to the pharmacy Web site at the completion of ordering its repeat prescriptions. The patient may be taken directly to a particular page of the pharmacy Web site, e.g. a home page, a special offer page etc.

A facility is also available for the Enigma processing centre 12 to handle e-mails, special information etc on behalf of other companies. Pharmacies are able to send information e.g. special offers to their respective patients, either through their own Web site 272 or on the Enigma Web site 250. A subscriber patient may access the Web site 250 from anywhere in the world where an access to the Internet 26 is available.

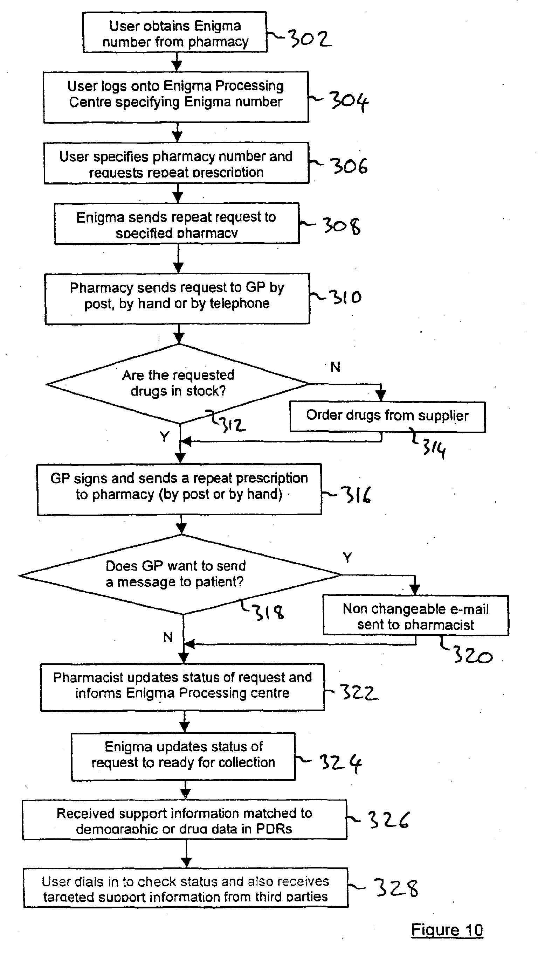

Referring now to Figure 10, a method of ordering a repeat prescription is now described. The method commences at Step 302 with the patient obtaining his or her Enigma number (PDR number) 180 from the pharmacy server 14. This may typically be provided to the patient on the Enigma card mentioned previously. The patient then logs on at Step 304 to the Web site 250 of the Enigma processing centre 12 and specifies the PDR number.

The patient then requests at Step 306 a repeat prescription and may specify the pharmacy code 166 for positively identifying the pharmacy. The Enigma processing centre 12 then sends at Step 308 the repeat prescription request to the specified pharmacy. On receipt of the request, the pharmacy updates its status to request received, prints out the request and then sends at Step 310 the request to GP 32 by post, by hand or in some cases the pharmacist can telephone the request through. At the same time, the stock control module 96 checks at Step 312 whether the requested drugs are in stock. If they are, a message is sent at Step 314 ordering the drugs from the supplier 34. The GP then processes the repeat prescription request. Namely, the GP 32 signs and returns at Step 316 a repeat prescription to the pharmacy by post or enables it to be collected by hand.

At this point, it is determined at Step 318 if the GP wishes to send a message to a patient? If a message is to be sent, then a non-changeable e-mail is sent at Step 320 to the pharmacist. If the GP did not want to send a message, or the message has been sent, the pharmacist receives the completed repeat prescription. At this stage, the Enigma processing centre is sent a message from the pharmacy server 14 at Step 322 indicating that the repeat prescription has been received from the GP. The Enigma processing centre updates its status accordingly. If the drugs are in stock or once they have been received as a result of an order, the pharmacist via the pharmacy server 14 then updates at Step 324 the status of the request to ready for collection or delivery and informs the Enigma processing centre 12.

The Enigma processing centre 12 then also updates at Step 324 the status of the request to ready for collection or delivery. The support information provided by the support information suppliers 36, is matched at Step 326 to PDRs 180 stored within the database 22. Finally, the patient dials in at Step 328 to the Web site 250 hosted on the Web server 50 of the Enigma processing centre 12 to check the status of the prescription or to access their PDR 180. At this moment, the patient also receives the targeted support information from any of the support information suppliers 36.

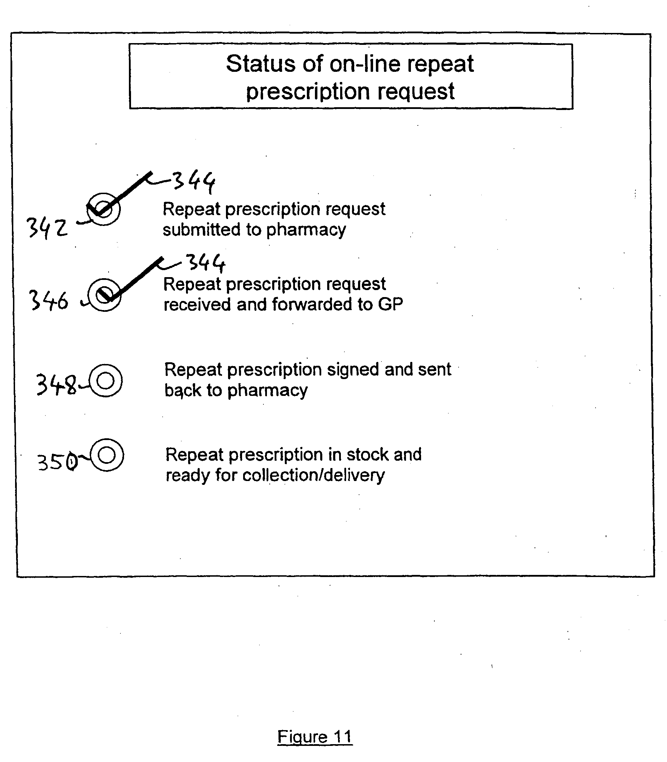

Referring now to Figure 11, a status screen 340 of the Web site 250 is now described. The status screen 340 shows four different status levels of the repeat prescription procedure 300 which are reached in the progression of the procedure.

The first status level 342 is highlighted with an easily recognisable mark 344 when the repeat prescription request has been submitted to the pharmacy server 14 by the Enigma processing centre 12. The second status level 346 is highlighted with the mark 344 when the repeat prescription request has been received by the pharmacy server 14 and has been forwarded to the patient's GP 32. The third status level 348, which has not yet been highlighted with the mark 344, indicates (when highlighted) that the repeat prescription has been signed (authorised) by the GP 32 and has been sent back to pharmacy server 14. The fourth status level 350 indicates when highlighted that the repeat prescription is in stock and is ready for collection or for delivery.

Accordingly, a patient logging onto the Web site of the Enigma processing centre 12 would be able to determine quickly from consideration of the marked status levels of the status screen 340 exactly how far his or her request has progressed down the repeat prescription process 300.

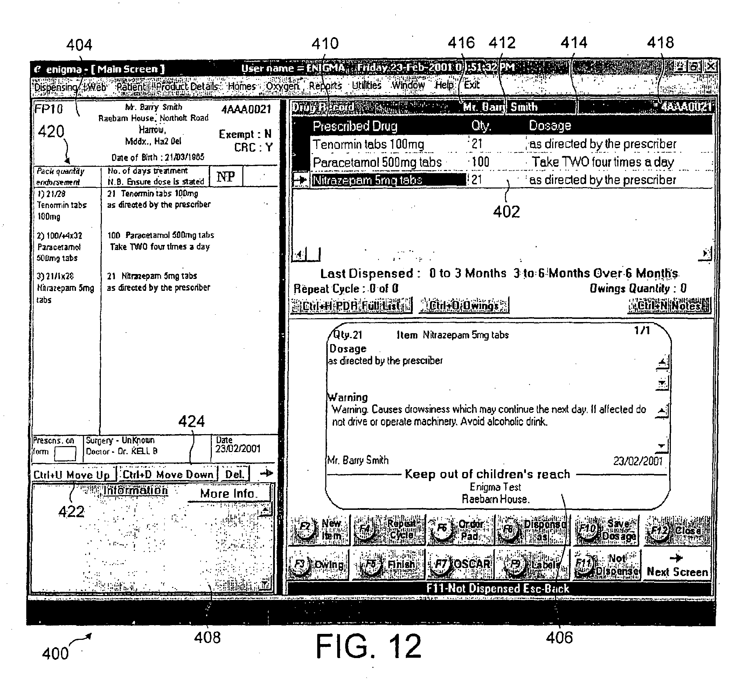

Referring to Figure 12, there is now described a GUI 400 used on the pharmacy server 14 to improve the pharmacists dispensing experience. The GUI 400 comprises four interactive display portions: a PDR portion 402, a prescription endorsement portion 404, a label viewing portion 406 and a general information portion 408.

The PDR portion 402 sets out key information from the PDR 180 on a line by-line basis. Each line specifies one of the items to be dispensed, indicating the prescribed drug 410 the quantity 412 and the dosage 414. As the PDR 180 is linked to the patient record 160 at the pharmacy server 14, the PDR portion 402 also displays the name of the patient 416 and the patient's PDR number 418.

The reason for providing such a detailed PDR representation on screen is to make the data entry for repeat prescriptions and the associated paperwork very simply. Rather than retyping in all of the details of a repeat prescription, or a new prescription, the information in the PDR 180 or in a drugs list (not shown) can be used to select the types of drugs required. Then only the quantity and dosage needs to be specified if it is different to that which has previously been specified.

Selection of the elements of the prescription in this way means that a high degree of automation can be attained by using the selected information to create the an endorsement section of the prescription hereinafter referred to as the 'OSCAR' (ON SCREEN GRAPHICAL REPRESENTATION) as shown in the endorsement portion 404 and the required labels for each of the medicines to be dispensed as shown in the label viewing portion 406.

The OSCAR 404 is generated from information extracted from the patient record 160 and PDR 180, together with some information obtained from the drugs database 144. All of the data presented on the OSCAR 404 is editable such that adjustments can be made on-screen prior to acceptance of the content of the endorsement. In order to complete the information required for filling out the OSCAR 404 of the endorsement FP10 form, the GUI 400 also retrieves from pack data (in the drugs database 144) the packet size used for dispensing the practitioner-specified amount of drug. For example, Figure 12 shows a first item of 21 tablets. However, the endorsement section 420 of the OSCAR 404 shows that 21/28 tablets were prescribed (21 tablets from a pack of 28) such that the actual remuneration to be paid to the pharmacist (pro-rata cost of the 21 tablets from a packet of 28 tablets) can be readily determined. Different pack sizes have different prices and as such the pack size is very important in determining the exact cost of the prescription to the pharmacist. Also as the pack sizes change from time to time as do the endorsement rules specified by the Government, the facility to edit the pack size specified in the OSCAR 404 is highly advantageous. Other endorsement rules are provided to facilitate the generation of the appropriate data for meeting Government requirements for meeting payment of the prescription.

The editing facility extends to manipulation of the positioning of various items in the OSCAR 404 to reflect exactly the order on the paper prescription generated by the GP 32. More specifically, the OSCAR 404 has a MOVE UP button 422 for moving a selected item up the order shown in the endorsement section 420 of the paper prescription representation OSCAR 404 and also a corresponding MOVE DOWN button 424 for moving the selected item down the order.

In practice, the information required for populating the OSCAR 404 has often been gathered together on receipt of a request for a repeat prescription. This is because the contents of the repeat prescription have already been specified by the patient or nursing home representative in making the request. This means that when the pharmacist comes to entering the repeat prescription created by the GP 32, identification of the patient means is all that is required to place all of the required information automatically in the OSCAR 404 and in the PDR 180 for editing if necessary by the pharmacist. This speeds up the data entry procedure.

The general information portion 408 enables any warnings relating to drug interactions for example to be displayed to the pharmacist.

Once the OSCAR 404 has been edited and the items in the endorsement section 420 match the items in the paper prescription, the original prescription can placed in the printer 106 and the endorsement section printed to reflect the on screen information. The prescription endorsed with this information is them sent to the NHS for processing and payment.

Every NHS prescription handled by the OSCAR 404 is reflected graphically within the pharmacy server 14 in respective and different colours. The PDR portion 402 also uses colour codes to help the pharmacist identify items in the PDR 180, which have been prescribed in different time periods. For example, the following colour scheme could used:

| | |
|---|---|
| Last Dispensed | Yellow colour |
| Items dispensed in last 3 months | Green colour |
| Items dispensed in last 6 months | Pink colour |
| Items dispensed more than six months | Black Colour |

Further detailed information on how a pharmacist interacts with the GUI 400 and how the pharmacy server 14 is used in dispensing is now set out below.

### Information displayed during dispensing

The following details are displayed on the PDR portion 402 to the pharmacist during dispensing:

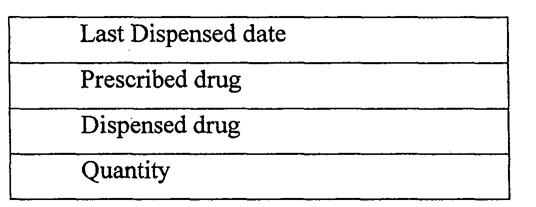

When the pharmacist selects the items, the GUI displays the details of the dispensing:

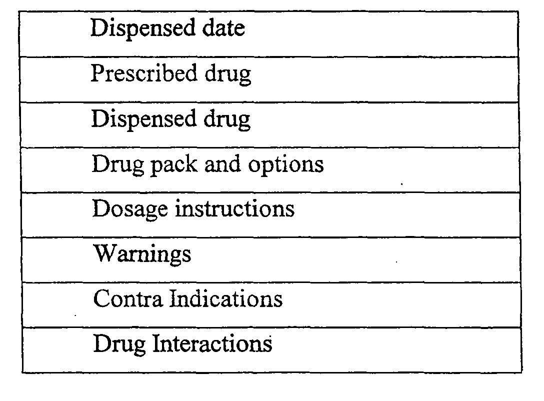

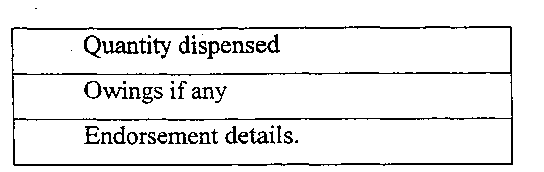

### Dispensing NHS Prescriptions

The dispensing process of a prescription takes place in the following sequence:
Select Prescription Type;
Select Patient Detail;
Confirm Prescriber;
Select Drug as Prescribed;
Select Drug as Dispensed;
Label;
Add Endorsement Options; and
Reorder Product.

### Each of these is now described in detail below:

### Select Prescription Type

The default is FP10 and GP10 in Scotland. The same procedure also applies to other NHS prescriptions.

### Select Patient Details

The search for the patient and his/her PDR is done on either the Enigma number, or Date of Birth, Surname/Initials. (**Note**: The DOB is in format DDMMYYYY). Where no PDR exists or a PDR cannot be found, the patient is treated as a 'new' patient and a new patient record 160 is created appropriately. For a new patient the record is created ensuring that it is unique. As more information is entered for the patient the pharmacy server 14 prompts for all patients still having a match. The pharmacist is forced to select one of those patients, until sufficient data has been entered so that is no match is left and a new record is created.

### Confirm Prescriber

When the pharmacist selects the GP, GP details are displayed in the OSCAR 404 in the GUI 400. In cases where the prescription has not been issued by the patient's regular GP, the pharmacist can change the prescriber to conform with the prescription at this point. However this will not change the patient's regular GP in the system. For every new patient the prescriber (as recorded in the prescription) must exist in the GP database 140. If the prescriber does not exist then a new record must be created in the GP database 140.

### Select Drug as Prescribed

- **For items previously prescribed** - the pharmacist selects it from the patient's PDR 180. The PDR 180 is shown on the screen and details of the items, which correspond to the items on the current form, can be transferred to the OSCAR 404. The pharmacist can edit them (e.g. Quantity, Dosage etc) as appropriate until the pharmacist is satisfied that the OSCAR accurately represents the contents of the current prescription.
- **For new items** - (i.e. not prescribed before) the pharmacist selects the items from the main drugs database 144 after having first input the quantity prescribed. This information which is followed by a build up of the Dosage and the Warnings are displayed in dispensing Label format in the label portion 406 of the GUI 400.
- Items are selected from the main drugs database 144 using Alpha characters with narrowing techniques. When a drug is selected for the very first time, the GUI 400 automatically applies an editable favourites code which consists of the first 3 characters of the Drug Name as it appears in the drugs database 144 plus the next available character.

### Select Drug as Dispensed

- If the item has a number of 'dispensed' possibilities then the GUI 400 prompts for all valid possibilities for endorsement and reordering using the Identifier and the Category Codes.
- The pharmacist is able to change the order of the drugs as displayed on the endorsement section 420 of the OSCAR 404 so that they correspond to the order of items as they appear on the prescription form. This is required, as the endorsements have to be added alongside the items to which they apply.
- Throughout the dispensing process the pharmacy server 14 checks the DIs, Warnings and Flashes for the prescribed drug and displays results in the general information portion 408.
- The above procedure continues until all items have been processed and the pharmacist is satisfied that the OSCAR 404 accurately reflects the prescription, when the GUI 400 prompts for label printing and endorsement of the prescription.

### Print Labels

- The GUI 400 also displays the default number of labels to be printed, which can be changed by selecting the label number on the label displayed or through a Function key.
- Once the labelling is complete, the information is transferred to the OSCAR 404 and the PDR 180. The pharmacist inserts the correct sequence and then adds any endorsement options.

### Add Endorsement Options

- The pharmacist can change the endorsement options by selecting the endorsement button or a Function Key.
- The endorsement can be done from the drug selection area. After endorsement is complete the GUI 400 switches back to the PDR and displays labels for the drug in card format. The pharmacist can view other labels by clicking on the card.
- The pharmacist is also be able to select an unendorsed prescription by entering the prescription number. This facility is useful for MDS, WEB prescriptions and electronic prescriptions (see alternative embodiment) from the GPs.
- After endorsing all the items on the OSCAR 404, the GUI 400 provides a facility for printing endorsements on the prescription.
- The Pharmacist can't edit or delete any item if the endorsement printing is over. However, the pharmacist can reprint an endorsement if required.

### Reorder Product

- The pharmacy server 14 normally reorders products as they are consumed according to Stock Control rules specified in the stock and suppliers database 150.
- If the pharmacist does not have the required quantity of the drug that is to be dispensed then a record of the balance owed to the patient is stored in an Owings register (not shown). It is also recorded and displayed in the patient's PDR 180 so that it can be dispensed later.
- There are EDI interfaces to the key suppliers for electronic ordering
- The GUI 400 enables the pharmacist to intervene, if required, and change the order details prior to transmission.

### Dispensing Other Prescriptions

### MDS Prescriptions

The pharmacy server 14 is able to handle dispensing for patients who are registered at nursing homes. The dispensing is the same as for other NHS prescriptions processing except that the drugs are dispensed against Medicine Administration Request (MAR) charts and the prescriptions follow subsequently and that the drugs are packed in and labelled in specific ways.
- The pharmacy server 14 is able to identify groups of patients for a nursing home, and display the list on screen and/or print off the list.
- An OSCAR 404 which will look like an FP10 is created for each patient from the MAR requests.
- Dispensing is according to NHS prescriptions and done from the MAR charts.
- Endorsement is done at a separate time in batches when the prescriptions are later received. A facility is provided for the pharmacist to select and endorse the unendorsed prescriptions.

### OTC (Over The Counter) Sales

The pharmacy server 14 provides a facility where occasionally, a patient may buy an OTC medicine and the pharmacist may want to add the purchase to the patient's PDR and to check that there are no interactions with his/her prescribed medicines. In this case:
- An OTC on the OSCAR looks like an FP10 with a grey colour background.
- Selection of items can be done through the PDR or by adding a new drug which is allowed for OTC dispensing (Restriction Field in Drug database).
- Dispensing is according to the pharmacist - a screen will display price of the drug.
- An invoice is printed at the end of the dispensing.
- No endorsement is done.

### Private Prescriptions

The pharmacy server 14 is also able to dispense Private Prescriptions. The forms do not have a standard shape or size. The process is similar to normal prescriptions in that drugs are supplied as stated on the prescription, but the difference is that there are no endorsements required. The full cost of the drugs is recovered from the patient.
- Private prescriptions on the OSCAR 404 look like an FP10 with a white colour background.
- All drugs irrespective of 'D' prefix and discontinued drugs are available for selection.
- Selection of items can be done through the PDR or by adding a new drug.
- Dispensing is done according to the prescription - the screen displays price of the drug.
- An invoice is always printed at the end of the dispensing.
- No endorsement is done.

### Old Prescriptions

The pharmacist can view old prescriptions by giving the prescription number and the pharmacy server 14 is arranged to look up the old prescription and display it. A facility is also provided for the pharmacist selecting any unendorsed prescriptions.

In summary, the OSCAR 404 displays the drugs that are going to be dispensed and is updated as the Pharmacist types in the quantity, prescribed drug that he reads from the paper prescription and the dosage that has to be put onto the label for the medicine. This is also written on the paper prescription. e.g. Take 1 three times a day. The OSCAR 404 also displays the "endorsement" which is the invoice to the government from the Pharmacist. It is printed when the prescription is completed. This is a visual check for the Pharmacist before completion so that it may amended if incorrect. The OSCAR 404 also displays the name of the patient, and their GP. By clicking on this area of the display the Pharmacist is presented with patient detail screen or change GP screen and thereby making the OSCAR interactive.

Identification of the correct type of NHS prescription is important since each prescriber prescribes only from a restricted list of products. Each prescription form is printed on different coloured paper for ease of recognition, which is also reflected graphically within the OSCAR 404 of the GUI 400.

There are several types of NHS prescriptions, which are issued by a number of authorised prescribers:

| **Prescriber** | **Colour of Prescription** |
|---|---|
| FP10 - Community doctor. (GP) | Pale green |
| FP10(D) - Dentist | Pale yellow |
| FP 10(N) - Community nurse | Grey |
| FP 10(PN) - Practice nurse | Lilac |
| FP10(HP) - Hospital Doctor | Pale Orange |
| FP10(L) - Locum | Green |

The overwhelming proportions of prescriptions are FP10s.

Additionally pharmacists may also dispense Private Prescriptions that may come from Doctors, Vets and Dentists. These do not have a standard shape or size and are graphically represented in the Pharmacy System as a blank form having the same shape and size as an FP 10.

The GUI 400 automatically allocates and prints a unique number for each prescription processed by the pharmacy server 14. The GUI 400 is able also to print this number on the label if required.

Processing takes place in a precise and clear manner. As pharmacists have peak dispensing periods during the day that usually coincide with local surgery hours, instantaneous access to the databases is critical to maintain prescription throughput and reduce in the shop waiting time for patients. Delays, whilst acceptable for producing reports at quieter times, are not acceptable during front line processing. Details of the prescription are input step-by-step starting with identification of the type of prescription being dispensed.

### Endorsement Procedure

These rules apply only to NHS prescriptions.

### Items to be endorsed

Usually all the items on the prescription need to be endorsed and all endorsement information is printed on the endorsement part of the form alongside each item. If no endorsement is necessary, then only the item number and '-' are to be printed. If the endorsed button (or endorsement function key) is pressed, the pharmacy server 14 endorses all items on the prescription otherwise the pharmacy server 14 endorses items whenever required. For example, if 4 items are on the prescription, and only 2 items need endorsement, then the pharmacy server 14 will only endorse those items. For items, which can't be dispensed, the endorsement text 'ND' will be printed for them. A unique number is printed on each prescription form and stored in the PDR for retrieval in the event of a dispute.

Whilst endorsing the Prescription, the Short name, Pharmacy PPA number, and date are printed.

Whilst not explicitly stated above, the feature of identifying patient's groups is available. The system is able to identify groups of patients, and display the list on screen and/or print off the list. In particular, it is useful to produce lists for patients of a nursing/residential home, patients of a particular GP or practice or patients with a particular illness. Patients must give this information when registered with Enigma. If patients are not registered with Enigma, but willing to give details, the pharmacist can collect the details at the time of creating the PDR 180 for the first time.

Also the Enigma processing centre 12 together with its linked pharmacy servers 14 are accessible by managers of chains of pharmacies. Managers can obtain reports of pharmacy performance, drugs prescribed, etc. based on data generated by each of the designated pharmacies which has been uploaded to and compiled by the processing centre 12. This gives managers unprecedented up to the moment information on pharmacy performance using real-time data which provides a visual appreciation of current situations.

It is to be appreciated that in an alternative embodiment of the present invention, the communications between the pharmacy server 14 and the GP 32 are carried out wholly electronically. This is not permissible in the UK at present under Government regulations but with the impending advent of the Electronic Transmission of Prescriptions (ETP) initiative, this is likely to come into force. In the alternative embodiment, the steps of the pharmacist sending the repeat prescription request 310 and the GP sending the repeat prescription 316 are carried out electronically. The advantage of this is that the amount the pharmacist has to key into the GUI 400 is reduced even further thereby assisting in the processing of ever increasing numbers of prescriptions.

Having described a particular preferred embodiment of the present invention, it is to be appreciated that the embodiment in question is exemplary only and that variations and modifications such as will occur to those possessed of the appropriate knowledge and skills may be made without departure from the spirit and scope of the invention as set forth in the appended claims. For example if further security was required it would be possible for the Enigma processing centre to have no patient details stored thereon at all. According the patients database 142 would only be provided at the patient's pharmacy server 14. Also, rather than the OSCAR 404 printing the compiled endorsement data and then printing the same on an original prescription, it is possible, subject to government regulations, for the compiled data to be sent as an electronic record to the NHA for payment processing. In this case the actual prescription could simply be sent as a confirmation that the endorsement data was correct without holding up payment for the pharmacist.

## Claims

1. A prescription reordering system (12) for requesting remote repeat prescriptions (182) for patients by way of a multistage, multiparty repeat prescription re-ordering process which includes a prescription authorisation stage involving a third party, the system comprising:
communications means (50, 52, 54) arranged to receive a request for a repeat prescription via a data network (18, 26), to enable patient selection of one of a plurality of known pharmacies, to forward the request to the patient-specified pharmacy (14), and to receive a corresponding response from the pharmacy regarding the allowance of the request;
storage means (22) for storing the response to the request from the pharmacy (14) for patient consideration; and
status indication means (62, 340) arranged to be updated by the patient-specified pharmacy (14) to indicate to the patient the current position of the request along the multistage repeat prescription process and to specify at least when a request has been submitted to the pharmacy (14), when the request has been sent to the third party (surgery) for authorisation and when the prescription (182) is ready for delivery or collection.

2. A system (12) according to Claim 1, wherein the communications means (50, 52, 54) is arranged to receive a request for a status check by a patient and to transmit the current status as determined by the status indication means (340) to the patient.

3. A system (12) according to Claim 1 or 2, wherein the communications means (50, 52, 54) is arranged to transmit and receive communications via a wide area telecommunications network.

4. A system (12) according to any preceding claim, further comprising means (22, 126) for storing a plurality of personal patient drugs records (180).

5. A system (12) according to Claim 4, wherein the patient drugs records (180) comprise a patient's demographic profile (186).

6. A system (12) according to Claim 4 or 5, wherein the communications means (50, 52, 54) is arranged to allow pharmacists access to patient drugs records (180).

7. A system (12) according to Claim 6, wherein the communications means (50, 52, 54) is arranged to enable the pharmacist to edit a particular patient drugs record (180) associated with that pharmacy (14).

8. A system (12) according to any of Claims 4 to 7, wherein the status of a given repeat prescription request is stored in a database record linked to a corresponding patient's patient drugs record (180).

9. A system (12) according to any preceding claim, wherein the communication means (50, 52, 54) comprises a security server (58) for preventing unauthorised access to the patient data records (180).

10. A system (12) according to any preceding claim, wherein the communications means (50, 52, 54) comprises a dedicated telephone exchange (16) connectable to the pharmacy via a public switched telephone network (18).

11. A system (12) according to any preceding claim, wherein each patient has a unique identifier (162) associated therewith and the communication means (50, 52, 54) is arranged to receive messages for specific patients identified by way of their unique identifier (162).

12. A system (12) according to any preceding claim, wherein the status indication means (62) is arranged to generate a simple indication of all the different stages of a prescription ordering process, and to highlight those stages which have occurred in the progression of a repeat prescription (182).

13. A system (12) according to any preceding claim, further comprising a database (128) of patient drug interactions and means (74) for checking a patient drugs record (180) against the database (128) for drug interactions.

14. A system (12) according to Claim 13, wherein the drug interactions checking means (74) is arranged to link together all patient drug records (180) belonging to a single patient into a consolidated drugs record and to check the patient's drug interaction against the consolidated drugs record.

15. A system (12) according to any preceding claim, further comprising a database (120) of pharmacy identifiers and a search engine (66) for searching the database (120) of pharmacy identifiers to find a specific pharmacy (14) registered with the system (12).

16. A system (12) according to Claim 15, wherein the database (120) of pharmacy identifiers (162) comprises personal identification information relating to each pharmacy including its name, address and contact details.

17. A system (12) according to any preceding claim, further comprising means (64) for generating and issuing a batch of the unique pharmacy-assignable identifiers (162) to each pharmacy (14).

18. A system (12) according to any preceding claim, further comprising construction means (78) arranged to enable an authorised user to construct a medicine administration chart for a patient, the construction means being arranged to permit access the patient drugs records (180) stored in the database (126) and to allow selection of items in the patient drugs record (180) for use in the medicine administration chart.

19. A combination of a patient information management system (12) according to any preceding claim and a pharmacy processing means (14) connectable via a communications network (16, 18, 26) to the communication means (50, 52, 54) for receiving and processing the request from the system (12) and notifying the system when the prescription (182) is ready for supply.

20. A combination according to Claim 19 as dependent from Claim 4 or any of Claim 5 to 18 as dependent from Claim 4, wherein each of the plurality of patient drugs records (180) comprises a unique pharmacy-assigned identifier (162) and the pharmacy processing means (14) comprises a database (24) of patient drugs records each having a unique identifier (162) corresponding to the unique identifier stored in the storing means (22, 126) of the system (12).

21. A combination according to Claim 19 or 20, wherein the pharmacy processing means (14) comprises means for receiving a repeat prescription request and stock level means (96) arranged to considering a stock list to determine the availability of a stock item specified in the request, wherein the stock level means (96) is arranged to place an order for the stock item if, after meeting the request, the amount of the stock item will fall below a predetermined level.

22. A combination according to any of Claims 19 to 21, wherein the communications means (50, 52, 54) comprises a dedicated telephone exchange (16) connected to the pharmacy processing means (14) and the communication means of the system (12) via a public switched telephone network (18).

23. A combination according to any of Claims 19 to 21, wherein the communications means (50, 52, 54) is arranged to support transmission of messages between the pharmacy processing means (12) and the communications means (50, 52, 54).

24. A combination according to Claim 23, further comprising message originating means (32) for generating a message from a general practitioner, the message originating means (32) being arranged to send the message to the communication means (50, 52, 54) via the pharmacy processing means (14), the message being secure against any editing at the pharmacy processing means (14).

25. A method of requesting remote repeat prescriptions for patients by way of a multistage, multiparty repeat prescription re-ordering process which includes a prescription authorisation stage involving a third party, the method comprising:
receiving a request for a repeat prescription via a data network (18, 20, 26),
enabling patient selection of one of a plurality of known pharmacies (14),
forwarding the request to the patient-specified pharmacy (14),
receiving a corresponding response from the pharmacy (14) regarding the allowance of the request;
storing the response to the request from the pharmacy for patient consideration; and
updating status indication means (62) with information supplied by the patient-specified pharmacy (14) to indicate to the patient the current position of the request along the multistage repeat prescription process, and to specify at least when a request has been submitted to the pharmacy (14), when the request has been sent to the third party (surgery) for authorisation and when the prescription (182) is ready for delivery or collection.

26. A computer program comprising program instructions for causing a computer to perform the method of Claim 25.
